# EUROPEAN PATENT APPLICATION

(11) **EP 1 956 081 A1**
(43) Date of publication of application: **13.08.2008**
(21) Application number: 06810651.7
(22) Date of filing: 27.09.2006
(51) Int. Cl.: C12N 5/10, A61K 35/12, A61P 7/06, A61P 35/00, A61P 35/02, A61P 37/00, C12Q 1/02, C12N 15/09, C12R 1/91

(54) **METHOD FOR DETECTION OF HUMAN PRECURSOR T CELL AND PRECURSOR B CELL**

(30) Priority: 30.09.2005 JP 2005286289
(71) Applicant: Nihon University, Tokyo 102-8275 (JP); Riken, Wako-shi, Saitama 351-0198 (JP)
(72) Inventor: MUGISHIMA, Hideo, Tokyo 102-8275 (JP); KATSURA, Yoshimoto, Tokyo 102-8275 (JP); KATO, Maiko, Tokyo 102-8275 (JP); KAWAMOTO, Hiroshi, Yokohama-shi Kanagawa 230-0045 (JP)
(74) Representative: Wakerley, Helen Rachael
(86) International application number: PCT/JP2006/319186
(87) International publication number: WO 2007/040123

(57) **Abstract**

[PROBLEMS] To provide a method for detection of a precursor T cell or precursor B cell, a method for evaluation of the property of a hematopoietic precursor cell in a source for transplantation, and a kit for use in the evaluation.

[MEANS FOR SOLVING PROBLEMS] A precursor T cell or precursor B cell can be detected by co-cultivating a stromal cell line with a monocyte. By using the detection method, a precursor T cell or precursor B cell can be quantified and can also evaluate the property of a hematopoietic precursor cell in a source for transplantation.

## Description

### Technical Field

To treat blood disorders such as intractable leukemia and severe aplastic anemia, a congenital immunodeficiency, or an inborn error of metabolism such as Hurler's disease, transplantation of hematopoietic stem/precursor cells is the most effective means.
Also, the transplantation of hematopoietic stem/precursor cells is important for blood forming and reconstruction of immune functions after chemotherapy with high doses of drugs for malignant tumors. Sources of the hematopoietic stem/precursor cells used are bone marrow, cord blood (hereinafter, referred to as CB), peripheral blood induced by a granulocyte colony stimulatory factor (hereinafter, referred to as G-CSF), etc. Of those, the CB has the advantage of no pain and no risk for a donor compared to the bone marrow and peripheral blood produced by G-CSF and is often used in a recent transplantation therapy.

Evaluation of the differentiation ability of hematopoietic stem/precursor cells contained in the transplantation sources is important for the success and failure of the transplantation. At present, the evaluation is performed only by an analysis of colony-forming cell culture using a methylcellulose semisolid medium (in vitro colony formation; hereinafter, referred to as CFU-C (colony-forming unit in culture)). However, in the method, only the abilities of hematopoietic stem/precursor cells to differentiate into erythrocyte, granulocyte/macrophage, or megakaryocyte lineage can be detected and the ability to differentiate into lymphocyte lineage is not detected. In the transplantation of hematopoietic stem/precursor cells, not only reconstruction of erythrocytes, leucocytes, and platelets but also reconstruction of immune functions are important. For the reconstruction of immune functions, it is important to transplant a sufficient amount of precursor cells capable of differentiating into T cells and B cells. However, an effective and simple method of examining the abilities of human hematopoietic stem/precursor cells to differentiate into T-cell lineage and B-cell lineage has not been established yet. Therefore, detection and quantification of precursor T cells and precursor B cells contained in a transplantation source has not been performed. Accordingly, a development of a practical and simple method of detecting and quantifying precursor T cells and precursor B cells is desired.

The abilities of human hematopoietic stem/precursor T cells to differentiate into T cells can be detected in principle by coculture with the fetal thymus gland of a mouse. It has been reported that use of the fetal thymus gland of an SCID mouse can improve the detection efficiency (see, for example, Non-patent Document 1). However, the culture system of the fetal thymus gland of a mouse is not quantitative and is complex, so it is practically impossible to use the system in clinical fields.
It has been clarified that Notch-Delta interaction is important for early differentiation of T cells. A recent report has revealed that differentiation of CD34⁺CD38⁻ lineage marker negative⁻ (Lin⁻) cells derived from human CB into CD4⁺CD8⁺ cells can be induced by culturing the cells on a stromal cell line OP9 where mouse delta-like 1 (Dll1) is forcibly expressed (see, for example, Non-patent Document 2). However, this method is not practical as a test method because it is performed using a fraction of CD34⁺CD38⁻Lin⁻ cells, which are considered as hematopoietic stem/precursor cells, collected using a cell sorter. It is necessary to establish a culture method capable of detecting and quantifying precursor T cells by culturing nucleated cells contained in a transplantation source without further fractionating the cells in the same way as the analysis of CFU-C. To establish the method, it is essential to distinguish T cells produced from hematopoietic stem/precursor cells by culture from T cells present before culture.

Meanwhile, differentiation of human hematopoietic stem/precursor cells into B cells can be achieved by coculture with a stromal cell line derived from mouse bone marrow. Previous reports revealed that differentiation into B-cell lineage can be induced by coculturing hematopoietic stem/precursor concentrated cell fractions, such as CD34^{high}Lin⁻cells, CD34⁺CD38⁻ cells, and CD34⁺CD38⁻CD7⁺ cells with a stromal cell line derived from mouse bone marrow (see, for example, Non-patent Documents 3, 4, 5, and 6). However, in order to simply quantify hematopoietic stem/precursor cells capable of differentiating into B cells contained in a transplantation source, it is necessary to establish a culture method including culturing nucleated cells contained in a transplantation source without further fractionating the cells in the same way as the analysis of CFU-C to calculate a precursor B cell number. To establish the method, it is necessary to determine such a culture condition that B cells contained in a nucleated cell layer cannot survive and differentiation of hematopoietic stem/precursor cells into B-cell lineage is supported.
Non-patent Document 1: Yeoman H, Gress RE, Bare CV, et al., Proc Natl Acad Sci USA. 1993 Nov 15; 90(22):10778-82
Non-patent Document 2: Ross N. La Motte-Nohs, Elaine Herer, and Juan Carlos Zuniga-Pflucker., BLOOD, vol. 105, Num. 4, 15, February, 2005.
Non-patent Document 3: DiGiusto DL, Lee R, Moon J, et al., Blood 1996; 87(4):1261-71.
Non-patent Document 4: Hao QL, Smogorzewska EM, Barsky LW, et al., Blood 1998; 91(11):4145-51
Non-patent Document 5: Crooks GM, Hao QL, Petersen D, et al., J Immunol 2000; 165(5):2382-9
Non-patent Document 6: Hao QL, Zhu J, Price MA, et al, Blood 2001; 97 (12): 3683-90

### Disclosure of the Invention

### Problems to be solved by the Invention

An object of the present invention is to provide a method for detection of precursor T cells or precursor B cells. A further object of the present invention is to provide a method for evaluation of the properties of hematopoietic precursor cells in a transplantation source using the detection method and to provide a kit for evaluating the transplantation source.

### Means for solving the Problems

The inventors of the present invention have made extensive studies to solve the above-mentioned problems, and as a result, the inventors have completed the present invention. That is, a stromal cell line is cocultured with a monocyte (hereinafter, referred to as MNC) to induce differentiation into T cells or B cells, to thereby yield CD5⁺CD7⁺CD3⁻ immature T cells or CD19⁺sIgM⁻ immature B cells. Then, the inventors have found out that the precursor T cells or precursor B cells can be detected by analyzing the resultant cells, thus completing the present invention.
The inventors of the present invention have established a cell obtained by forcibly expressing human Delta-like 1 (DLL1) in a stromal cell line TSt-4 (hereinafter, referred to as TSt-4). The cell where DLL1 is forcibly expressed or a cell where Dll1 is forcibly expressed can be used for detecting precursor T cells contained in a transplantation source or the like. Meanwhile, TSt-4 can be used for detecting precursor B cells. Moreover, the inventors of the present invention have established a method of quantifying precursor T cells or precursor B cells to evaluate the properties of hematopoietic precursor cells in a transplantation source based on the detection method.

That is, the present invention relates to the following items (1) to (22):
(1) a method for detection of a precursor T cell or a precursor B cell, including coculturing a stromal cell line with a monocyte to induce differentiation into a T cell or a B cell;
(2) a method for detection of a precursor T cell or a precursor B cell, including coculturing a stroma cell line with a CD34⁺ lineage marker negative' (Lin⁻) cell or a CD34⁺CD38⁻Lin⁻ cell contained in a monocyte to induce differentiation into a T cell or a B cell;
(3) a detection method according to Item (1) or (2), including culturing the monocyte, CD34⁺Lin⁻ cell, or CD34⁺CD38⁻Lin⁻ cell by a limiting dilution method;
(4) a detection method according to any one of Items (1) to (3), including analyzing the cell obtained by the induction of differentiation using a flow cytometer;
(5) a method for detection of a precursor T cell or a precursor B cell according to any one of Items (1) to (4), including analyzing an immature T cell of CD5⁺CD7⁺CD3⁻ or an immature B cell of CD 19⁺sIgM⁻ using a flow cytometer;
(6) a method for detection of a precursor T cell according to any one of Items (1) to (5), in which the stromal cell line is a stroma cell line TSt-4 where DLL1 or Dll1 is forcibly expressed;
(7) a method for detection of a precursor B cell according to any one of Items (1) to (6), in which the stromal cell line is a stroma cell line TSt-4;
(8) a detection method according to any one of Items (1) to (7), in which the monocyte, CD34⁺Lin⁻ cell, or CD34⁺CD38⁻Lin⁻ cell is derived from a transplantation source;
(9) a detection method according to Item (8), in which the transplantation source is at least one selected from cord blood, peripheral blood, and bone marrow;
(10) a method for quantification of precursor T cells or precursor B cells including culturing cells in the detection method according to any one of (1) to (9) by a limiting dilution method, analyzing the cells proliferated during the culture; and determining a ratio of cells where appearance of precursor T cells or precursor B cells is not detected;
(11) a method for evaluation of property of a hematopoietic precursor cell in a transplantation source including quantifying the precursor T cells or precursor B cells detected in a detection method according to any one of Items (1) to (9);
(12) a method for evaluation of property of a hematopoietic precursor cell in a transplantation source according to Item 11, which is used for determining frequency of precursor T cells or precursor B cells per monocyte;
(13) a method for evaluation of the property of a hematopoietic precursor cell in a transplantation source according to Item (11) or (12), further comprising quantifying erythrocyte and granulocyte lineage precursor cells by in vitro colony formation;
(14) a transplantation source classified depending on a disease to be treated after evaluating property of a hematopoietic precursor cell by the method according to any one of Items (11) to (13);
(15) a method for selection of transplantation source classified depending on a disease to be treated after evaluating the property of a hematopoietic precursor cell by a method according to any one of Items (11) to (13);
(16) a kit for evaluating a transplantation source using the detection method according to any one of Items (1) to (9);
(17) a kit for evaluating a transplantation source according to Item (16), including a stromal cell line;
(18) a kit for evaluating a transplantation source according to Item (17), wherein the stromal cell line is a stroma cell line TSt-4 where DLL1 or Dll1 is forcibly expressed;
(19) a stroma cell line TSt-4 where DLL1 is forcibly expressed;
(20) a stroma cell line TSt-4 where DLL1 is forcibly expressed by an introduction of a gene;
(21) a stromal cell line TSt-4 where DLL1 is forcibly expressed according to Item (20), in which the gene introduced is a DLL1 gene; and
(22) a stromal cell line TSt-4 where DLL1 is forcibly expressed according to Item (21), in which the accession number is FERM BP-10375.

### Effect of the Invention

If precursor T cells or precursor B cells are quantified by a method of detecting precursor T cells or precursor B cells of the present invention, properties of hematopoietic precursor cells in a transplantation source can be evaluated. This method can be used for previous examination of the availability of a transplantation source, which relates to the success and failure of the transplantation.

### Brief Description of Drawings

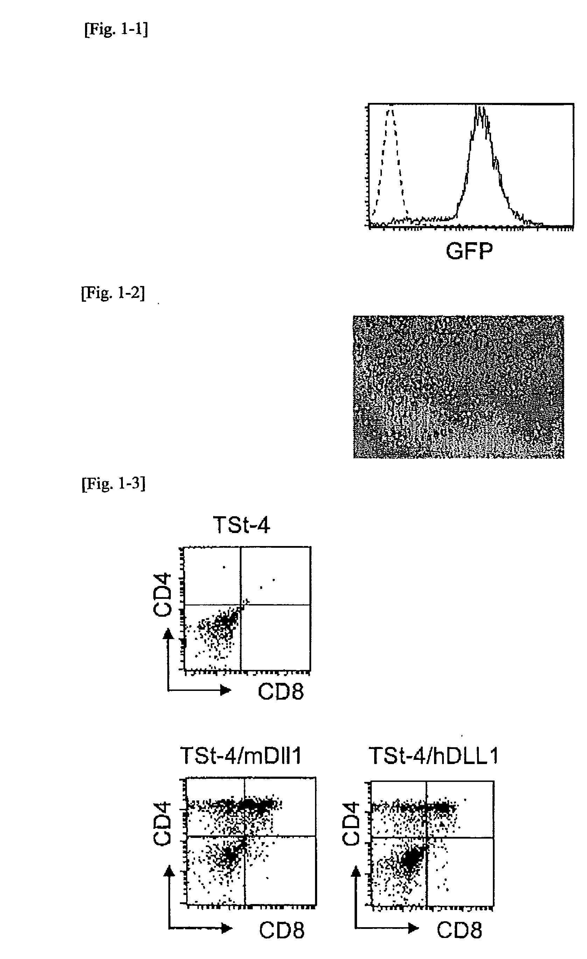

Fig. 1-1 shows that TSt-4/hDLL1 is produced by introducing DLL1 into TSt-4 (Example 1).
Fig. 1-2 shows a picture of colonies derived from mFTs cocultured with TSt-4/mDll1 (Example 1).
Fig. 1-3 shows CD4/8 profiles of cells (Example 1).
Fig. 2-1 shows proliferation of mature T cells (CD3⁺ cells) in coculture of TSt-4/hDLL1 with CBMNCs (Example 2).
Fig. 2-2 shows differentiation of precursor T cells in coculture of TSt-4/hDLL1 with CBMNCs (Example 2).
Fig. 3 shows FACS analyses of cells after coculture of CD34⁺CD38⁻Lin⁻ cells with TSt-4 or TSt-4/hDLL1 (Examples 3 and 7).
Fig. 4 shows the reconstruction of DJ in cells in coculture of CD34⁺CD38⁻Lin⁻ cells with TSt-4 or TSt-4/hDLL1 (Example 3).
Fig. 5-1 shows the ratios of wells where appearance of CD5⁺CD7⁺ T cells from CD34⁺CD38⁻Lin⁻ cells are not detected are plotted for each number of cultured cells (Example 3).
Fig. 5-2 shows the ratios of wells where appearance of CD3⁻CD5⁺ T cells from MNCs are not detected are plotted for each number of cultured cells (Example 4).
Fig. 6 is shows the frequency stability in subculture of TSt-4/hDLL1 (Example 5).
Fig. 7 is shows disappearance of CD19⁺ B cells in MNCs in coculture of TSt-4 with CBMNCs (Example 6).
Fig. 8 is shows collection of CD34⁺CD38⁻Lin⁻ cells and CD34⁺Lin⁻ cells (Examples 2 and 6).
Fig. 9-1 shows the ratios (%) of wells where no CD34⁺Lin⁻ cells differentiate into B cells are plotted for each number of cultured cells (Example 7).
Fig. 9-2 shows the ratios (%) of wells where no CD34⁺CD38⁻Lin⁻ cells differentiate into B cells are plotted for the numbers of cultured cells (Example 7).
Fig. 10 shows the ratios (%) of wells where appearance of CD19⁺ B cells from MNCs are plotted for each number of cultured cells (Example 8).
Fig. 11 shows the frequency stability of TSt-4 by subculture (Example 9).

### Best Mode for carrying out the Invention

The "stromal cell line" of the present invention is not particularly limited as long as it is a cell capable of inducing differentiation of a precursor T cell contained in MNCs into an immature T cell by coculture with MNC or a cell capable of inducing differentiation of a precursor B cell into an immature B cell. Moreover, the stromal cell line is preferably a cell capable of detecting a precursor T cell or a precursor B cell contained in MNC by inducing differentiation into an immature T cell or an immature B cell by the differentiation induction.
In the case of detecting precursor T cells, "a stromal cell line TSt-4 where DLL1 or Dll1 is forcibly expressed" is particularly preferably used. On the other hand, in the case of detecting precursor B cells, "a stroma cell line TSt-4" is particularly preferably used. The species of an animal that is used for producing those stromal cell lines is not particularly limited.

The "stromal cell line TSt-4 where DLL1 or Dll1 is forcibly expressed" is a cell where DLL1 or Dll1 is forcibly expressed, which is produced by introducing a gene of DLL1 or Dll1 of a Notch ligand into "a stromal cell line TSt-4" established from the thymus gland of a mouse.
The gene to be introduced may be the whole or part of the DLL1 gene or Dll1 as long as it has a function of DLL1 or Dll1. The species of an animal that is used for producing those genes is not particularly limited. Examples thereof include a mouse gene-introduced stromal cell line where Dll1 is forcibly expressed (hereinafter, referred to as TSt-4/mDll1) and a human gene-introduced stromal cell line where DLL1 is forcibly expressed (accession number: FERM BP-10375, hereinafter, referred to as TSt-4/hDLL1).
In the method of detecting precursor T cells, differentiation of precursor T cells contained in MNCs into CD5⁺CD7⁺CD3⁻ immature T cells can be induced by coculturing the TSt-4/mDll1 or TSt-4/hDLL1 with MNCs or CD34⁺CD38⁻Lin⁻ cells contained in MNCs. On the other hand, in the method of detecting precursor B cells, differentiation of precursor B cells contained in MNCs into CD19⁺ B cells can be induced by coculturing the "stromal cell line TSt-4" with MNCs, or with CD34⁺Lin⁻ cells or CD34⁺CD38⁻Lin⁻ cells contained in MNCs.

The method of detecting precursor T cells of the present invention is carried out by detecting precursor T cells contained in MNCs by coculturing a stromal cell line with MNCs or with CD34⁺CD38⁻Lin⁻ cells contained in MNCs. Specifically, differentiation of precursor T cells contained in MNCs or CD34⁺CD38⁻Lin⁻ cells contained in MNCs into CD5⁺CD7⁺CD3⁻ immature T cells is induced by coculturing TSt-4/mDll1 or TSt-4/hDLL1 with MNCs. Subsequently, the CD5⁺CD7⁺CD3⁻ immature T cells obtained by the differentiation induction are analyzed using a flow cytometer or the like. In the analysis, if the CD5⁺CD7⁺CD3⁻ immature T cells produced by the differentiation induction are detected, precursor T cells are confirmed to be present in MNCs or in CD34⁺CD38⁻Lin⁻ cells contained in MNCs. As a result, precursor T cells can be detected. Meanwhile, in the case of detecting and quantifying precursor T cells, monocytes are preferably cultured by the limiting dilution method.
In coculture of the present invention, precursor T cells contained in MNCs cannot differentiate into a mature T cell that expresses CD4/CD8, and therefore, it is possible to distinguish mature T cells that are originally present from mature T cells proliferated during the culture. Therefore, precursor T cells can be quantitatively detected by coculturing MNCs with TSt-4/hDLL1 without fractionating CD34⁺CD38⁻Lin⁻ cells that are considered to as hematopoietic stem/precursor cells.

The method of detecting precursor B cells of the present invention is carried out by detecting precursor B cells contained in MNCs by coculturing a stromal cell line with MNCs or with CD34⁺Lin⁻ cells or CD34⁺CD38⁻Lin⁻ cells contained in MNCs. Specifically, differentiation of precursor B cells contained in MNCs into CD19⁺ B cells is induced by coculturing TSt-4 with MNCs or with CD34⁺Lin⁻ cells or CD34⁺CD38⁻Lin⁻ cells contained in MNCs. Subsequently, the CD19⁺ B cells obtained by the differentiation induction are analyzed using a flow cytometer or the like. In the analysis, if the CD19⁺ B cells produced by the differentiation induction are detected, precursor B cells are confirmed to be present in MNCs or CD34⁺Lin⁻ cells or CD34⁺CD38⁻Lin⁻ cells contained in MNCs. Meanwhile, in the case of detecting and quantifying precursor B cells, monocytes are preferably cultured by the limiting dilution method.
In the coculture of the present invention, CD19⁺ B cells that are originally present in MNCs disappear, and therefore, all the CD19⁺ cells that are newly produced by the differentiation induction are derived from precursor B cells. Accordingly, precursor B cells can be quantitatively detected by coculturing MNCs with TSt-4 without fractionating MNCs, CD34⁺Lin⁻ cells, or CD34⁺CD38⁻Lin⁻ cells that are considered to as hematopoietic stem/precursor cells. All the CD19⁺ B cells that are originally present in MNCs are sIgM⁺, while all the CD19⁺ cells that are newly produced by the differentiation induction are sIgM⁻ immature B cells. The fact shows that all the CD19⁺ cells newly obtained by the differentiation induction are derived from precursor B cells.

The "method of evaluating the property of a hematopoietic precursor cell in a transplantation source" of the present invention can be carried out by using the method of detecting a precursor T cell or a precursor B cells of the present invention for MNC contained in a transplantation source to be evaluated.
Specifically, the numbers of precursor T cells and precursor B cells contained in a transplantation source are measured by detecting immature T cells or immature B cells by the detection method of the present invention. This method can be used to count the numbers of hematopoietic precursor cells in different transplantation sources and to evaluate the properties of the transplantation sources.
The property of a transplantation source can be evaluated based on the frequency of precursor T cells or precursor B cells per monocyte. For example, the evaluation can be performed according to the Poisson distribution or the like by detecting precursor T cells by an analysis of immature T cells obtained by differentiation induction from monocytes cultured by the limiting dilution method. Then, the number of precursor T cells is detected to determine the frequency of precursor T cells.
The "method of evaluating the property of a hematopoietic precursor cell in a transplantation source" of the present invention may further include quantifying erythrocyte and granulocyte lineage precursor cells by in vitro colony formation. This method can be used to examine the ability of a transplantation source to differentiate into erythrocyte and granulocyte lineage cells and the ability of a transplantation source to differentiate into precursor T cells or precursor B cells and to comprehensively grasp the property of the transplantation source.

The "transplantation source" of the present invention may include all sources that may be used for the transplantation, and examples thereof include CB, peripheral blood, and bone marrow. Meanwhile, the "transplantation source classified depending on a disease to be treated" is a transplantation source that has a property evaluated in a detection method of the present invention and is classified into, for example, a transplantation source containing many precursor T cells, a transplantation source containing many precursor B cells, and a transplantation source containing many precursor T and B cells, which are classified for a disease to be treated in consideration of the property. For example, a transplantation source containing many precursor T and B cells may be classified whose "a disease to be treated" are severe combined immunodeficiency and Wiskott-Aldrich syndrome, which are immunodeficiency diseases that cause a disorder in both of the T cells and B cells.

The "kit for evaluating a transplantation source" of the present invention is a kit including at least two of reagents and cells for evaluating the property of a hematopoietic precursor cell in a transplantation source in combination. Examples thereof include: a kit including a plate and a medium for cocultivation in combination; and a kit including a plate for cocultivation where TSt-4, TSt-4/mDll1, or TSt-4/hDLL1 is cultured as a stromal cell line.
Hereinafter, the present invention will be described in more detail by way of examples, but it is not limited to the examples.

### Example 1

### <Establishment of stromal cell line>

TSt-4 derived from the thymus gland of a mouse, which had been established by Watanabe et al. (reference), was used. Culture of TSt-4 was performed using, as a complete medium, RPMI 1640 (Sigma-Aldrich, St. Louis, MO) supplemented with 5% fetal bovine serum (FBS; Lot. 511042; BioSource International Camarillo, CA), 1 mM sodium pyruvate (Wako Pure Chemical Industries, Osaka, Japan), 1 mM non-essential amino acid solution (Invitrogen), 5 × 10⁻⁵ M 2-mercaptoethanol (2-ME; NACARAI TESQUE, Osaka, Japan), 100 µg/mL streptomycin, and 100 U/mL penicillin.
Dll1 was introduced into TSt-4 using a retrovirus vector pMSCV-IRES-EGFP (MIE vector) (from Dr. Nagahiro Minato, Kyoto University) (pMSCV-Dll-1-IRES-EGFP), to thereby yield TSt-4/mDll1. The sequence of the introduced Dll1 gene is described in SEQ ID NO: 1 in the sequence list. Meanwhile, in the same way as above, DLL1 was introduced (pMSCV-DLL1-IRES-EGFP), to thereby yield TSt-4/hDLL1. The sequence of the introduced DLL1 gene is described in SEQ ID NO: 2 in the sequence list.

Introduction of MIE vector (hereinafter, referred to as MIE) obtained by introducing DLL1 (Dll1) into TSt-4 was performed in accordance with the following method. That is, Phoenix cells (from Dr. Koichi Ikuta, Kyoto University: prepared by Dr. Toshio Kitamura, Tokyo University), serving as packaging cells, were prepared at 8 × 10⁵ cells/2 mL using 10% FCS-supplemented DMEM (Sigma D5796) as a medium and inoculated into a collagen-coated plate (IWAKI #4810-010) at 2 mL/well. The cells were cultured overnight under conditions of 37°C and 5% CO₂.
Before introduction of a gene, a medium supplemented with 25 µM chloroqine was prepared, heated to 37°C, and used for exchanging the medium for the Phoenix cells. Subsequently, a DNA solution was prepared according to the composition described in Table 1. The DNA solution was added to 250 µL of 2 × HEPES buffer saline (HBS) in another FACS tube while foaming the solution, to thereby yield 500 µL of a solution. The resultant solution was evenly added dropwise to the culture plate for the Phoenix cells. The Phoenix cells were cultured again under conditions of 37°C and 5% CO₂, and 5 hours later, small cell populations were detected in the cultured cells. 8 hours after the beginning of the culture, 2 mL of the medium was exchanged, and after a lapse of 24 hours, 1 mL of the medium was exchanged. After exchanging the media, the cells were cultured overnight, and the culture supernatant was collected. Then, the supernatant was sterilized by membrane filtration using a 0.22-µm filter (MILLIPORE), and 10 pg of polygrene was added thereto. The culture supernatant after addition of polygrene was exchanged for the supernatant of confluent TSt-4 cells prepared in advance in a 12-well plate (Costar). Centrifugation was performed at 32°C and 1,750 G for 1 hour, and 1 mL of the complete medium was added, followed by culture under conditions of 37°C and 5% CO₂ to proliferate TSt-4 cells.

**[Table 1]**

| Composition of DNA solution | |
|---|---|
| CaCl₂ | 25 µL |
| Mf D.W.* | 220 µL |
| MIE(1 µg/µL) | 5 µL |
| Total | 250 µL |

| | |
|---|---|
| *Mf D.W. (membrane filtration D.W.): Distilled water sterilized by membrane filtration. | |

For the respective cells introduced with the genes, the excitation of green fluorescence protein (GFP) caused by a 488 nm laser was detected to confirm the introduction of the genes. As shown in Fig. 1-1, the left side of the graph shows negative cells where no gene was introduced, while the right side shows positive cells where a gene was introduced. Most cells are shown on the right end, and it was found that cells introduced with a gene were obtained at a high rate. Only the cells introduced with a gene were separated using a cell sorter and cultured. Among them, TSt-4/hDLL1 (Accession No; FERM BP-10375) was deposited at the International Patent Organism Depositary, National Institute of Advanced Industrial Science and Technology.
Reference Document: Watanabe Y, Mazda O, Aiba Y, Iwai K, Gyotoku J, Ideyama S, Miyazaki J, Katsura Y., Cell Immunol., 142(2), 385-97, Jul., 1992.

### <Confirmation of differentiation-inducing ability of stromal cell line>

Mouse fetal thymocytes (hereinafter, referred to as mFTs) were cocultured with TSt-4/mDll1 or TSt-4/hDLL1, and the cultured cells were analyzed to examine the differentiation-inducing abilities of the stromal cell lines. As a control, the mFTs were cocultured with TSt-4.
RPMI 1640 supplemented with 10% FCS, 1 mM sodium pyruvate, 1 mM non-essential amino acid solution, 5 × 10⁻⁵ M 2-ME, 100 µg/mL streptomycin, and 100 U/mL penicillin was used as a complete medium. 4 days before the beginning of culture, TSt-4, TSt-4/mDll1, or TSt-4/hDLL1 was inoculated with the complete medium into each well of a 12-well plate. On the monolayer cells, 300 mFT double negative (DN) cells, where both of CD4 and CD8 of mFTs were not expressed, were inoculated to perform coculture. Fig. 1-2 shows cobble stone-like colonies formed in the coculture of TSt-4/mDll1 with DN cells.
The thymus gland was removed from a C57BL/6 mouse (CLEA Japan) fetus at day 13 of gestation, and mFTs were separated and inoculated on each confluent stromal cell line. Coculture was performed at 37°C in the presence of 5% CO₂, and after a lapse of 7 days, the cultured cells were separated with trypsin-EDTA (Invitrogen).
After the FcR blocking, the cells were stained with FITC (PE)-labeled anti-mouse CD8 (clone 53-6.7; Pharmingen) and FITC (PE)-labeled anti-mouse CD4 (clone H129.19; Pharmingen), and the cell surface markers were analyzed using a flow cytometer FACSCalibur (Nippon Becton Dickinson, Japan).

Fig. 1-3 shows CD4/8 profiles of the collected cells. The 300 mFT DN cells cocultured with TSt-4/mDll1 for 7 days were found to include not only DP (double positive) cells where both of CD4/CD8 were expressed but also many CD4 SP (single positive) cells where only CD4 was expressed. In this system, CD8 SP cells where only CD8 was expressed were rare. Similarly, in the 300 mFT DN cells cocultured with TSt-4/hDLL1 for 7 days, DP cells and CD4 SP cells were induced as in the case of TSt-4/mDll1. On the other hand, in the case of control, i.e., in the case of coculture with TSt-4, few DP and SP cells were detected.
These results revealed that TSt-4/mDll1 and TSt-4/hDLL1 of the present invention were effective for induction of differentiation of precursor T cells.

### Example 2

### Detection method of human precursor T cell

### <Principle>

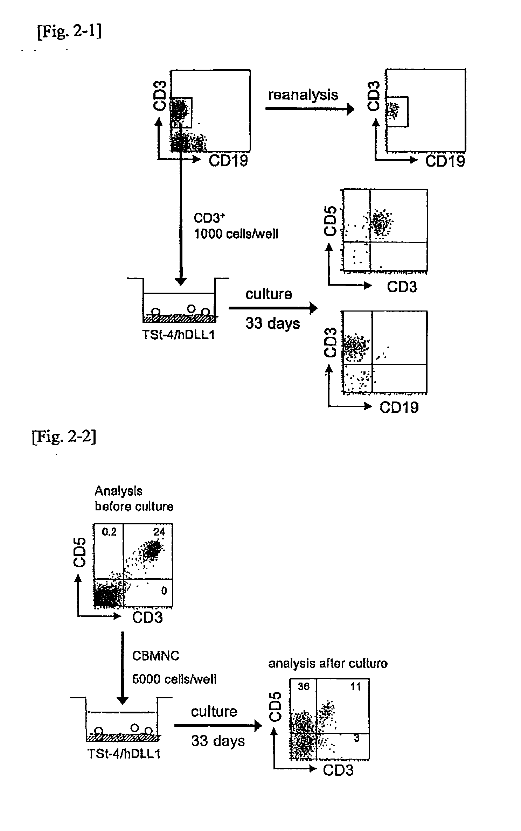

Precursor cells capable of differentiating into myeloid or erythrocyte lineage can be subjected to a clonal assay using CFU-C of cord blood monocytes without further treatment (hereinafter, referred to as CBMNCs), and the number of the cells can be determined with the assay. If hematopoietic stem/precursor cells capable of differentiating into T-cell lineage can be detected with CBMNCs to quantify the cells, it is possible to previously examine the T-cell producing ability of CB to be used.
Therefore, in the present invention, in order to detect precursor T cells contained in a transplantation source such as CB, TSt-4/hDLL1 established in the present invention was cocultured with CBMNCs to induce differentiation of precursor T cells contained in the CBMNCs into T cells.
In the coculture, TSt-4/hDLL1 inhibited the appearance of CD19⁺ B cells from human hematopoietic precursor cells and induced differentiation of precursor T cells into only CD3⁻CD5⁺ or CD3⁻CD5⁺CD7⁺ immature T cells. Cells detected after culture were CD19⁻CD3⁻CD5⁺CD19⁻, and therefore not be B cells. Meanwhile, few CD3⁻CD5⁺CD19⁻ immature T cells were present in MNCs before culture (Fig. 2-2), so it is possible to easily distinguish CD3⁻CD5⁺CD19⁻ immature T cells with an anti-CD3 and CD5 antibodies. Although mature T cells (CD3⁺ cells) contained in CBMNCs were proliferated in the coculture (Fig. 2-1), precursor T cells were proliferated into CD3⁻CD5⁺CD19⁻ immature cells, and therefore, it is possible to distinguish mature T cells that were originally present from T cells that were newly produced by differentiation/proliferation from the precursor cells.
Therefore, it is possible to count precursor T cells per CB by analyzing the frequency of appearance of CD3⁻CD5⁺ cells after coculture with TSt-4/hDLL1.

### <Preparation of sample>

To detect precursor T cells per MNC contained in CB, samples were prepared.

### 1. Preparation of CBMNC

There was used CB that was supplied from Tokyo Cord Blood Bank for research purposes and collected within 24 hours. MNCs were separated by specific gravity centrifugation using Lymphoprep (1.077 g/cm³) (AXIS-SHIELD PoC AS, Oslo, Norway). The cells were washed three times and dispensed in an amount of 1.5 × 10⁷ cells, and cryopreserved with Cell Banker (Juji Field, Tokyo, Japan) as CBMNCs.

### 2. Preparation of CD34⁺ cell and CBCD34⁺CD38⁻Lin⁻ cell

### 1) Preparation of CD34⁺ cell

In the case of coculture using CD34⁺CD38⁻Lin⁻ cells contained in CBMNCs, only CD34⁺ cells were prepared in advance to effectively sort CD34⁺CD38⁻Lin⁻ cells by a cell sorter. That is, CD34⁺ cells were prepared as follows: part of the CBMNCs separated above was treated with MiniMACS, MACS MS Separation Columns, and MACS Direct CD34 Progenitor Cell Isolation Kit (all Miltenyi Biotec, Bergisch, Gladbach, Germany) to produce CD34⁺ cells from the MNCs, and the CD34⁺ cells were cryopreserved with Cell Banker.

### 2) Preparation of CD34⁺38⁻Lin⁻ cell

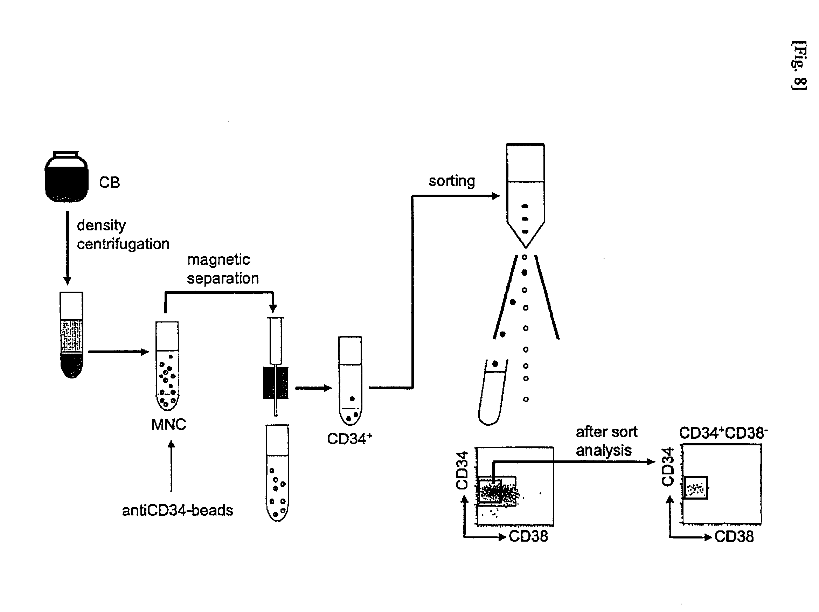

The CD34⁺ cells of CB preserved above were rapidly thawed and washed, followed by FcR blocking. The cells were stained with fluorescein isothiocyanate (FITC)-anti-lineage markers (Lin) [CD3 (clone HIT3a), CD4 (clone RPA-T4), CD5 (clone UCHT2), CD7 (clone M-T701), CD8 (clone HIT8a), CD14 (clone M5E2), CD19 (clone HIB19), CD56 (clone B159), Glycophorin A (clone Ga-R2 (HIR2))], PE(APC)labeled anti-human CD34 (clone 581), PE(APC)labeled anti-human CD38 (clone HIT2) (all Pharmingen), and CD34⁺38⁻Lin⁻ cells were collected by a flow cytometer FACSVantage (Nippon Becton Dickinson, Japan) (Fig. 8).

### Example 3

### <Detection and quantification of precursor T cell contained in CD34⁺CD38⁻Lin⁻ cell>

### 1. Coculture of CD34⁺CD38⁻Lin⁻ cell with TSt-4/hDLL1

CD34⁺CD38⁻Lin⁻ cells were cocultured with TSt-4/hDLL1 by the limiting dilution method. For comparison, CD34⁺CD38⁻Lin⁻ cells were cocultured with TSt-4. 4 days before the beginning of culture, TSt-4 or TSt-4/hDLL1 was inoculated with the complete medium into each well of a 48-well plate. CD34⁺CD38⁻Lin⁻ cells were rapidly thawed and washed, and the obtained cells were subjected to limiting dilution and inoculated on each confluent stromal cell line. The cells were cultured at 37°C and 5% CO₂ for about 33 days, and the medium was exchanged every one week in the culture period.

### 2. Analysis of cell after culture

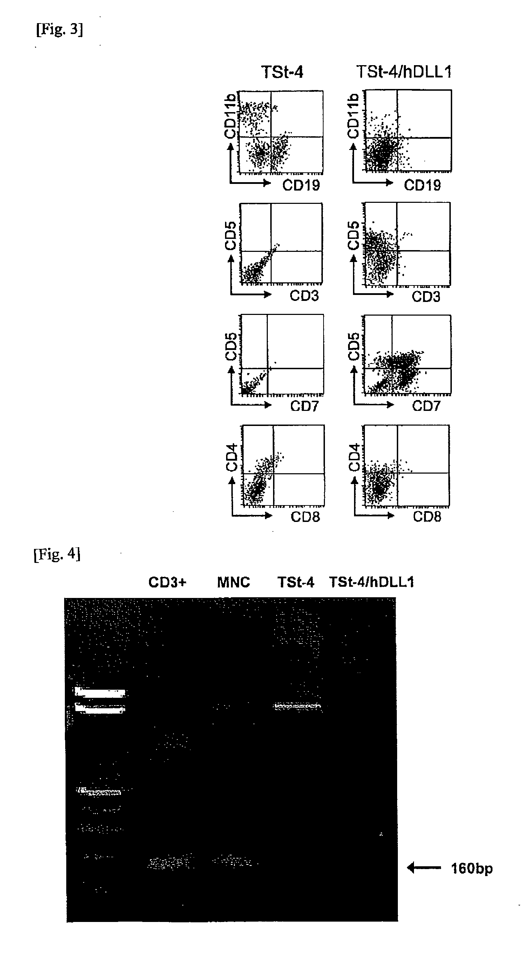

After the culture, the resultant cells were analyzed by flow cytometry.
The cells where differentiation was induced by the coculture were scraped off from the plate together with the stromal cell lines and subjected to FcR blocking, and the cells were stained with FITC-anti-CD3, FITC-anti-CD7, FITC-anti-CD8, FITC-anti-CD19, PE-anti-CD4 (clone RPA-T4 (Pharmingen)), PE-anti-CD5 (clone UCHT2 (Pharmingen)), PE-anti-CD11b (clone Bear1 (IOTest)), and APC-anti human-CD45 (clone J33 (IOTest)), followed by an analysis of the antigens on the cell surfaces using a flow cytometer FACSCaliber. In the analysis, APC-anti human-CD45 was used to remove GFP⁺ stromas.
The results of the flow cytometry are shown in Fig. 3. In the coculture of the CD34⁺CD38⁻Lin⁻ cells with TSt-4, myeloid lineage cells and B cells appeared, while T-lineage cells were not produced. On the other hand, in the coculture of CD34⁺CD38⁻Lin⁻ cells with TSt-4/hDLL1, differentiation into B-lineage cells did not occur, and CD5⁺ cells appeared. The cells were considered to be CD7⁺CD19⁻ and to be classified into T-lineage cells, but CD3, CD4, CD8, etc. were not expressed.

### 3. Analysis of reconstruction of TCR gene

A genomic DNA assay was used to analyze whether a DJ region in TCR β-chain was reconstructed or not. After the culture, 1 × 10⁵ cells were dissolved in 20 µL of a PCR buffer, and the solution was incubated at 95°C for 10 minutes and used as a PCR template.
Primers described in SEQ ID NOS: 3 and 4 in the sequence list were used. There were used 20 µL of a reaction solution, 4 µL of the template, 1.6 µL of 10 × PCR buffer, 1.6 µL of 2.5 mM dNTPs, 4 pmol of each primer, and 0.6 U of Taq polymerase. Thermocycling was performed as follows: 94°C for 5 minutes; 40 cycles of 94°C for 1 minute, 60°C for 1 minute, and 72°C for 1 minute; and 72°C for 10 minutes. PCR products were developed by electrophoresis on a 1.2% agarose gel.

If the CD5⁺ cells produced by the coculture with TSt-4/hDLL1 are T-lineage cells, the DJ region in TCR β-chain was considered to be reconstructed. Genomic DNA was analyzed by PCR using primers for the DJ region in TCR β-chain, described in SEQ ID NOS: 3 and 4 in the sequence list, and as a result, in cells produced by the coculture with TSt-4/hDLL1, a germ line disappeared, and a 160-bp band, which represents DJ reconstruction, was detected (Fig. 4). In the cells produced by the coculture with TSt-4, the band was not detected, and only the germ line was detected. The CD5⁺ cells produced from CD34⁺CD38⁻Lin⁻ cells by the coculture with TSt-4/hDLL1 were considered to be immature T cells.

### 4. Measurement of the number of precursor T cell in CD34⁺CD38⁻Lin⁻ cell

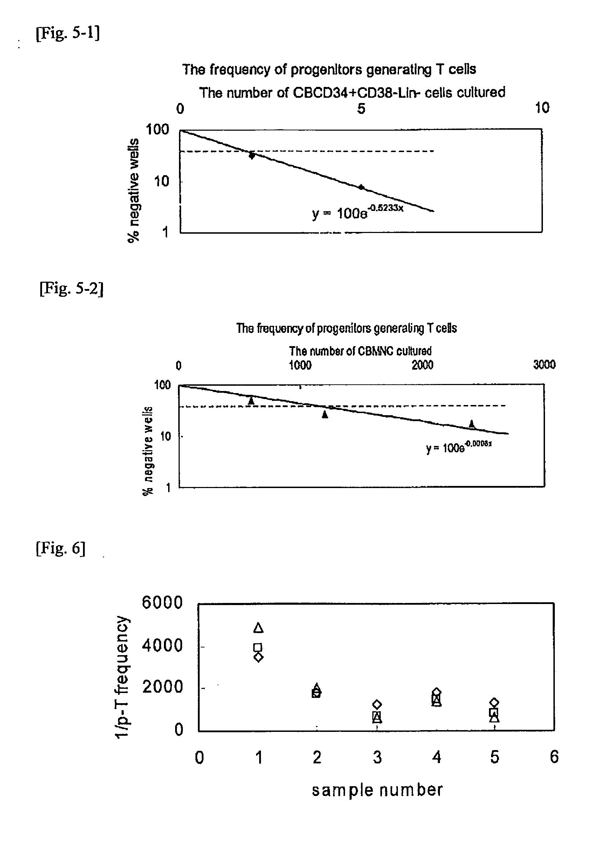

The frequency of appearance of CD5⁺CD3⁻ T cells in CD34⁺CD38⁻Lin⁻ cells was determined by coculture of the CD34⁺CD38⁻Lin⁻ cell subjected to limiting dilution with TSt-4/hDLL1. The ratio of wells where CD5⁺CD7⁺ cells were not detected was plotted for each number of cultured cells as a ratio of cells where CD5⁺CD7⁺ cells were not detected, and the results were analyzed according to the Poisson distribution (Fig. 5-1). The results suggested that precursor cells capable of differentiating into T cells were contained in CD34⁺CD38⁻Lin⁻ cells at a ratio of 1 : 1.9.

### Example 4

### <Detection and quantification of precursor T cell contained in CBMNC>

### 1. Coculture of CBMNC with TSt-4/hDLL1

CBMNCs were cocultured with TSt-4/hDLL1 by the limiting dilution method. 4 days before the beginning of the culture, TSt-4/hDLL1 was inoculated into each well of a 48-well plate with the complete medium. CBMNCs were rapidly thawed and washed, and the obtained cells were subjected to limiting dilution and inoculated on confluent TSt-4/hDLL1. The cells were cultured at 37°C and 5% CO₂ for about 33 days, and the medium was exchanged every one week in the culture period.

### 2. Analysis of cell after culture

After the culture, the resultant cells were analyzed by using a flow cytometry.
The cells where differentiation was induced by the coculture were scraped off from the plate together with the stromal cell line and subjected to FcR blocking, and the cells were stained with FITC-anti-CD3, PE-anti-CD5 (clone UCHT2 (Pharmingen)), and APC-anti human-CD45 (clone J33 (IOTest)), and the antigens on the cell surfaces were analyzed using a flow cytometer FACSCalibuer. In the analysis, APC-anti human-CD45 was used to remove GFP⁺ stromas.

### 3. Measurement of the number of precursor T cell in CBMNC

The frequency of appearance of CD5⁺CD3⁻ T cells in CBMNCs was determined by coculture of the CBMNCs subjected to limiting dilution with TSt-4/hDLL1. As described above, few immature T cells of CD5⁺CD3⁻ were present in MNCs before culture, and therefore the cells can be easily distinguished by using anti-CD3 and CD5 antibodies. The ratio of wells where CD5⁺CD3⁻ cells were not detected was plotted for the numbers of cultured cells as a ratio of cells where CD5⁺CD3⁻ cells were not detected, and the results were analyzed according to the Poisson distribution (Fig. 5-2). The results revealed that precursor cells capable of differentiating into T cells were contained in CBMNCs at a ratio of 1 : 1,205.
The CBMNCs used above was found to contain 0.18% CD34⁺CD38⁻Lin⁻ cells, and from the result, the number of precursor T cells were calculated based on the ratio of precursor T cells contained in CD34⁺CD38⁻Lin⁻ cells determined in Example 3 above (precursor T cells were found to be contained in CD34⁺CD38⁻Lin⁻ cells at a ratio of 1 : 1.9). As a result, the precursor T cells was found to be contained in MNCs at a ratio of 1 : 1,056, which was about the same ratio as that of the culture in MNCs.

### Example 5

### 1. Confirmation of detection method of precursor T cell

To confirm stability of the cell differentiation-inducing ability of the coculture system of the present invention, five CBMNC samples were separately cultured three times to examine the frequencies of precursor T cells. The frequencies were found to be almost constant, and the culture system was estimated to be stable (Fig. 6). Meanwhile, differences in the frequencies of precursor T cells depending on CB were further examined. The examination was performed for ten CBMNC samples derived from different origins. As a result, the frequencies of precursor T cells were found to vary from 1/861 to 1/4,803, and the average of the frequencies was calculated to be 1/2,803 (Table 2).

### 2. Measurement of myeloid lineage precursor cell by CFU-C assay

CBMNCs (2 × 10⁴/mL) or CD34⁺38⁻Lin⁻ cells (100 cells/mL) were inoculated into MethoCult GFH4434V (Stem Cell Technology, Vancouver, BC; Lot.3H079061), and colonies were counted on day 14 of culture. In the culture of CBMNCs, CFU-GMs were detected in CBMNCs at an average ratio of 1 : 697. Moreover, in the culture of CD34⁺38⁻Lin⁻ cells, CFU-GMs were detected in CD34⁺38⁻Lin⁻ cells at an average ratio of 1 : 3.6.
In comparison of cord bloods derived from different origins, the ratios of the numbers of CFU-GMs to the numbers of precursor T cells are not constant and vary. Therefore, a combination of the results of measurement using CFU-C assay and the results of measurement of the number of precursor T cells can be used as a novel method of evaluating a transplantation source.

**[Table 2]**

| Number of samples | Frequency | | |
|---|---|---|---|
| | CFU-GM | Precursor T cell | CFU-GM/Precursor T cell |
| 1 | 1/448 | 1/2,986 | 6.67 |
| 2 | 1/408 | 1/1,679 | 4.12 |
| 3 | 1/1,198 | 1/4,803 | 4.00 |
| 4 | 1/922 | 1/3,004 | 3.25 |
| 5 | 1/455 | 1/1,291 | 2.84 |
| 6 | 1/760 | 1/1,845 | 2.43 |
| 7 | 1/1,538 | 1/3,513 | 2.29 |
| 8 | 1/380 | 1/861 | 2.27 |
| 9 | 1/922 | 1/1,808 | 1.96 |
| 10 | 1/667 | 1/1,278 | 1.92 |

| | | | |
|---|---|---|---|
| 1) CFU-GM: Precursor cells that formed colonies including granulocyte lineage cells and monocyte/macrophage lineage cells. | | | |

### Example 6

### Detection method of human precursor B cell

### <Principle>

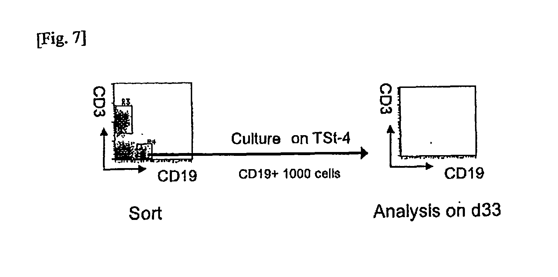

In the present invention, in order to detect precursor B cells contained in a transplantation source such as CB, TSt-4 was cocultured with CBMNCs to induce differentiation of precursor B cells contained in the CBMNCs.
In order to easily evaluate B-cell producing ability of a hematopoietic stem/precursor cell transplantation source, it is important to establish a method of detecting a hematopoietic stem/precursor cell capable of differentiating into B-cell lineage using MNCs without further treatment. In the present invention, the coculture was continued for 33 days. CD19⁺ B cells contained in MNCs were killed during the coculture (Fig. 7), and therefore all the CD19⁺ B cells that appeared after the coculture with TSt-4 were found to be derived from hematopoietic precursor cells. Therefore, even if CBMNCs contain B cells, it is possible to detect only B cells obtained by differentiation/proliferation from precursor B cells.
Accordingly, precursor cells per MNC in CB can be counted by analyzing the frequency of CD19⁺ B cells that appear after culture by coculture with TSt-4.

### <Preparation of stromal cell line and sample>

To detect precursor B cells per MNC in CB, samples were prepared.
1. Preparation of stromal cell line TSt-4 was used. For the cultivation of TSt-4, used were 5% Fetal Bovine Serum (FBS; Lot. 511042; Biosource International, Camarillo, CA), 1 mM sodium pyruvate (Wako Pure Chemical Industries, Osaka, Japan), 1 mM non-essential amino acid solution (Invitrogen), 5 × 10⁻⁵ M 2-mercaptoethanol (2-ME; NACARAI TESQUE, Osaka, Japan), 100 µg/ml streptomycin, and RPMI1640 (Sigma-Aldrich, St. Louis, MO) added with 100 U/ml Penicillin G.

### 2. Preparation of sample

There was used CB that was supplied from Tokyo Cord Blood Bank for research purposes and was collected within 24 hours. MNCs were separated by specific gravity centrifugation using Lymphoprep (1.077 g/cm³) (AXIS-SHIELD PoC AS, Oslo, Norway). The cells were washed three times and dispensed in an amount of 1.5 × 10⁷ cells, and the obtained cells were cryopreserved with Cell Banker (Juji Field, Tokyo, Japan) as CBMNCs.

### 3. Preparation of CD34⁺ cell

In the case of coculture using CD34⁺Lin⁻ cells or CD34⁺CD38⁻Lin⁻ cells contained in CBMNCs, CD34⁺ cells were prepared and preserved in advance by a simple method to effectively sort CD34⁺Lin⁻ cells or CD34⁺CD38⁻Lin⁻ cells by a cell sorter. That is, CD34⁺ cells were prepared as follows: part of the CBMNCs separated above was treated with MiniMACS, MACS MS Separation Columns, and MACS Direct CD34 Progenitor Cell Isolation Kit (all Miltenyi Biotec, Bergisch, Gladbach, Germany) to produce CD34⁺ cells from the MNCs, and the CD34⁺ cells were cryopreserved with Cell Banker (Juji Field, Tokyo, Japan).

### 4. Preparation of CD34⁺Lin⁻ cell or CD34⁺38⁻Lin⁻ cell

The CD34⁺ cells preserved above were rapidly thawed and washed, followed by FcR blocking. The cells were stained with fluorescein isothiocyanate (FITC)-anti-lineage markers (Lin) [CD3 (clone HIT3a), CD4 (clone RPA-T4), CD5 (clone UCHT2), CD7 (clone M-T701), CD8 (clone HIT8a), CD14 (clone M5E2), CD19 (clone HIB19), CD56 (clone B159), Glycophorin A (clone Ga-R2 (HIR2))], R-phycoerythrin (PE)-anti-CD34 (clone 581), Allophycocyanin (APC)-anti-CD38-allophycocyanin (clone HIT2) (all Pharmingen), and CD34⁺38⁻Lin⁻ cells and CD34⁺Lin⁻ cells were collected by a flow cytometer FACSVantage (Nippon Becton Dickinson, Japan) (Fig. 8).

### Example 7

### <Detection and quantification of precursor B cell contained in CD34⁺Lin⁻ cell or CD34⁺38⁻Lin⁻ cell>

### 1. Coculture of CBMNC, CD34⁺Lin⁻ cell, or CD34⁺38⁻Lin⁻ cell with TSt-4

CD34⁺Lin⁻ cells or CD34⁺38⁻Lin⁻ cells were cocultured with TSt-4. 4 days before the beginning of coculture, TSt-4cells were inoculated with the complete medium into each well of a 48-well plate. CD34⁺Lin⁻ cells or CD34⁺38⁻Lin⁻ cells were inoculated on confluent TS-4 cells according to the limiting dilution method. The cells were cultured at 37°C and 5% CO₂ for about 33 days, and the medium was exchanged every one week in the culture period.

### 2. Analysis of cell after culture

After the culture, the resultant cells were analyzed by using a flow cytometry.
The cells where differentiation was induced by the coculture were scraped off physically from the plate together with the stromal cell lines and subjected to FcR blocking, and the cells were stained with FITC-anti-CD3, FITC-anti-CD7, FITC-anti-CD8, FITC-anti-CD19, PE-anti-CD4 (clone RPA-T4 (Pharmingen)), PE-anti-CD5 (clone UCHT2 (Pharmingen)), PE-anti-CD11b (clone Bear1 (IOTest)), and APC-anti-CD45 (clone J33 (IOTest)), followed by an analysis of the antigens on the cell surfaces using a flow cytometer FACSCaliber (Becton Dickinson). In the analysis, CELLQuest (BD Biosciences) was used.
The results of the flow cytometry are shown in Fig. 3. In the coculture of CD34⁺Lin⁻ cells or CD34⁺38⁻Lin⁻ cells with TSt-4, differentiation into myelocytic cells (CD11b⁺) and B-lineage cells (CD19⁺) was detected. However, cells of T-cell lineage were not detected at all. That is, the results shows that TSt-4 supports the differentiation of CD34⁺38⁻Lin⁻ cells, which are hematopoietic stem/precursor cells in the earliest stage in human CB, into myeloid lineage cells and B cells.

### 3. Quantification of the number of precursor B cells contained in CD34⁺Lin⁻ cells or CD34⁺38⁻Lin⁻ cells

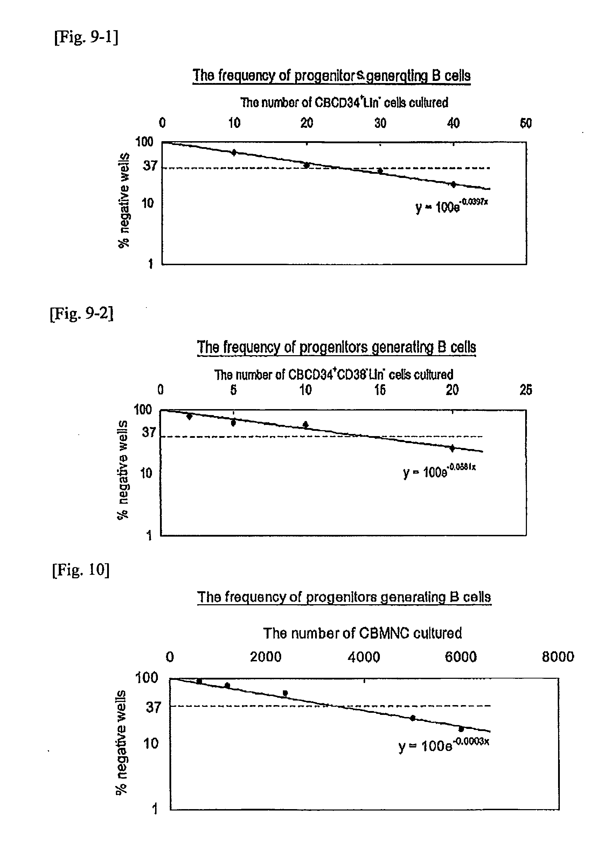

The frequency of appearance of B cells in CD34⁺Lin⁻ cells or CD34⁺38⁻Lin⁻ cells was determined by the coculture of the CD34⁺Lin⁻ cells or CD34⁺38⁻Lin⁻ cells subjected to limiting dilution with TSt-4. The ratio of wells where B cells were not detected was plotted for the each number of cultured cells (Figs. 9-1 and 9-2), and the results were analyzed according to the Poisson distribution. The results revealed that precursor cells capable of differentiating into B cells were contained in CD34⁺Lin⁻ cells at a ratio of 1 : 25.0 and in CD34⁺38⁻Lin⁻ cells at a ratio of 1 : 14.6.

### Example 8

### <Detection and quantification of precursor B cell contained in CBMNCs>

### 1. Coculture of CBMNCs with TSt-4

CBMNCs were cocultured with TSt-4 by the limiting dilution method. 4 days before the beginning of culture, TSt-4 cells were inoculated with the complete medium into each well of a 48-well plate. CBMNCs were rapidly thawed and washed, and the obtained cells were subjected to limiting dilution and inoculated on each confluent TSt-4 cell. The cells were cultured at 37°C and 5% CO₂ for about 33 days, and the medium was exchanged every one week in the culture period.

### 2. Analysis of cell after culture

After the culture, the resultant cells were analyzed by using a flow cytometry. The cells where differentiation was induced by the coculture were scraped off physically from the plate together with the stromal cell lines and subjected to FcR blocking, and the cells were stained with FITC-anti-CD19, PE-anti-CD11b (clone Bear1 (IOTest)), and APC-anti human-CD45 (clone J33 (IOTest)), followed by an analysis of the antigens on the cell surfaces using a flow cytometer FACSCaliber (Becton Dickinson). In the analysis, CELLQuest (BD Biosciences) was used.

### 3. Quantification of the number of precursor B cells in CBMNC

The frequency of appearance of CD19⁺ B cells in CBMNCs was determined by coculture of the CBMNCs subjected to limiting dilution with TSt-4. CD19⁺ cells contained in MNCs before culture disappear during the culture. Therefore, even if the MNCs are cultured without further treatment, it is possible to detect only B cells newly produced from precursor cells, thereby detecting precursor B cells contained in CBMNCs. The ratio of wells where CD19⁺ B cells were not detected was plotted for each number of cultured cells, and the results were analyzed according to the Poisson distribution (Fig. 10). The results revealed that precursor cells capable of differentiating into B cells were contained in MNCs at a ratio of 1 : 3,809.
The CBMNCs used above was found to contain 0.79% CD34⁺Lin⁻ cells, and from the result, the number of precursor B cells was calculated based on the ratio of precursor B cells contained in CD34⁺Lin⁻ cells determined in Example 7 above. As a result, the precursor B cells was found to be contained in CBMNCs at a ratio of 1 : 3,175, which was about the same ratio as that of the culture in MNCs.

### Example 9

### 1. Confirmation of detection method of precursor B cell

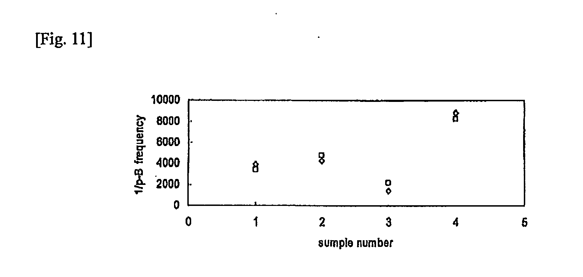

To confirm stability of the cell differentiation-inducing ability of the coculture system of the present invention, four CBMNC samples were separately cultured twice to examine the frequencies of precursor B cells. The frequencies in samples were found to be almost constant, and the culture system was estimated to be stable (Fig. 11). Meanwhile, differences in the frequencies of precursor B cells depending on CBs were further examined. The examination was performed for ten CBMNC samples derived from different origins. As a result, the frequencies of precursor B cells were found to vary from 1/765 to 1/12,585, and the average of the frequencies was calculated to be 1/889 (Table 3).

**[Table 3]**

| Number of samples | Frequency | | |
|---|---|---|---|
| | CFU-GM | Precursor B cell | CFU-GM/Precursor B cell |
| 1 | 1/448 | 1/9,653 | 21.5 |
| 2 | 1/760 | 1/12,585 | 16.6 |
| 3 | 1/1,198 | 1/10,357 | 8.65 |
| 4 | 1/667 | 1/4,267 | 6.40 |
| 5 | 1/1,538 | 1/8,799 | 5.72 |
| 6 | 1/922 | 1/5,047 | 5.47 |
| 7 | 1/455 | 1/2,449 | 5.38 |
| 8 | 1/922 | 1/3,930 | 4.26 |
| 9 | 1/408 | 1/1,445 | 3.54 |
| 10 | 1/380 | 1/765 | 2.01 |

| | | | |
|---|---|---|---|
| 1) CFU-GM: Precursor cells that formed colonies including granulocyte lineage cells and monocyte/macrophage lineage cells. | | | |

### 2. Measurement of myeloid lineage precursor cell by CFU-C assay

CBMNCs (2 × 10⁴/mL), CD34⁺ (100 cells/mL), or CD34⁺CD38⁻Lin⁻ cells (100 cells/mL) were inoculated into MethoCult GFH4434V (Stem Cell Technology, Vancouver, BC; Lot.3H079061), and colonies were counted on day 14 of culture. In the culture of CBMNCs, CFU-GMs were detected in CBMNCs at an average ratio of 1 : 697. Moreover, in the culture of in CD34⁺Lin⁻ cells, CFU-GMs were detected in CD34⁺Lin⁻ cells at an average ratio of 1 : 4.8, and in the culture of CD34⁺CD38⁻Lin⁻ cells, CFU-GMs were detected in CD34⁺38⁻Lin⁻ cells at an average ratio of 1 : 3.7 (Table 4).
The measurement method is mainly intended to measure granulocyte lineage and macrophage precursor cells. In comparison of cord bloods derived from different origins, the ratios of the numbers of CFU-GMs and the numbers of precursor B cells are not constant and vary. Therefore, a combination of the results of measurement using CFU-C assay and the results of measurement of the number of precursor B cells can be used as a novel method of evaluating a transplantation source.

**[Table 4]**

| Cell populations¹⁾ | Number of colonies | |
|---|---|---|
| | CFU-GEMM²⁾ | CEU-GM |
| CD34+Lin- | 1.0±0 | 21.0±1.3 |
| CD34+CD38-Lin- | 1.0±0.5 | 27.3±1.9 |

| | | |
|---|---|---|
| 1) Average and standard error in the case where 100 cells were cultured three times. 2) CFU-GEMM: Multipotent hematopoietic precursor cells that formed colonies including all blood cell lineage cells such as granulocyte lineage, monocyte/macrophage lineage, erythrocyte lineage, and megakaryocyte lineage cells. CFU-GM: Precursor cells that formed colonies including granulocyte lineage cells and monocyte/macrophage lineage cells. | | |

### Industrial Applicability

The method of detecting precursor T cells or precursor B cells of the present invention can be used to evaluate the properties of hematopoietic precursor cells in a transplantation source. This method is stable and simple, so the availability of a transplantation source can be examined in advance. Moreover, the method can be used for quality control of a transplantation source.

### Reference to deposited biological materials

A. Name and address of depository institution where biological materials of the present invention were deposited
Name: International Patent Organism Depositary, National Institute of Advanced Industrial Science and Technology
Address: Tsukuba Central 6, 1-1, Higashi 1-Chome, Tsukuba-shi, Ibaraki-ken, Japan
B. Date when the materials were deposited at the institution A July 15, 2005 (deposition date based on the Budapest Treaty)
C. Accession number given by the institute A for the deposition FERM BP-10375

INTERNATIONAL FORM

BUDAPEST TREATY ON THE INTERNATIONAL RECOGNITION OF THE DEPOSIT OF MICROORGANISMS
FOR THE PURPOSES OF PATENT PROCEDURE

RECEIPT IN THE CASE OF AN ORIGINAL DEPOSIT

issued pursuant to Rule 7.1 by the INTERNATIONAL DEPOSITARY AUTHORITY identified at
the bottom of this page.

TO DEPOSITOR:

Name: Hideo MUGISHIMA

Address: 4-23-11, Kugahara, Ota-ku, Tokyo 146-0085, Japan

I. IDENTIFICATION OF MICROORGANISM
Identification Reference Given
by the Depositor:
TSt-4/hDLL1 | Accession Number:

FERM BP-10375
II. SCIENTIFIC DESCRIPTION AND/OR PROPOSED TAXONOMIC POSITION
The microorganism identified under I above was accompanied by a document stating
the following item(s).
☑ Scientific Properties
☑ Taxonomic Position
III. RECEIPT AND ACCEPTANCE
This authority accepts the microorganism identified under I above, which was
received on July 15, 2005.
IV. RECEIPT OF TRANSFER
This authority received the microorganism identified under I above on .
Further, the authority received the transfer of the deposit based on the Budapest Treaty
on .
V. INTERNATIONAL DEPOSITARY AUTHORITY
Name: International Patent Organism Depositary
National Institute of Advanced Industrial Science and Technology
Representative: Dr. Masakazu Yamaoka, Director (Sealed)

Address: AIST Tsukuba Central 6,
1-1, Higashi 1-chome, Tsukuba-shi,
Ibaraki-ken 305-8566, JAPAN

Date: July 15, 2005

## Claims

1. A method for detection of a precursor T cell or a precursor B cell, comprising coculturing a stromal cell line with a monocyte to induce differentiation into a T cell or a B cell.

2. A method for detection of a precursor T cell or a precursor B cell, comprising coculturing a stroma cell line with a CD34⁺ lineage marker negative⁻ (Lin⁻) cell or a CD34⁺CD38⁻Lin⁻ cell contained in a monocyte to induce differentiation into a T cell or a B cell.

3. A detection method according to Claim 1 or 2, comprising culturing the monocyte, CD34⁺Lin⁻ cell, or CD34⁺CD38⁻Lin⁻ cell by a limiting dilution method.

4. A detection method according to any one of Claims 1 to 3, comprising analyzing the cell obtained by the induction of differentiation using a flow cytometer.

5. A method for detection of a precursor T cell or a precursor B cell according to any one of Claims 1 to 4, comprising analyzing an immature T cell of CD5⁺CD7⁺CD3⁻ or an immature B cell of CD 19⁺sIgM⁻ using a flow cytometer.

6. A method for detection of a precursor T cell according to any one of Claims 1 to 5, wherein the stromal cell line is a stroma cell line TSt-4 where human Delta-like 1 (DLL1) or mouse delta-like 1 (Dll1) is forcibly expressed.

7. A method for detection of a precursor B cell according to any one of Claims 1 to 6, wherein the stromal cell line is a stroma cell line TSt-4.

8. A detection method according to any one of Claims 1 to 7, wherein the monocyte, CD34⁺Lin⁻ cell, or CD34⁺CD38⁻Lin⁻ cell is derived from a transplantation source.

9. A detection method according to Claim 8, wherein the transplantation source is at least one selected from cord blood, peripheral blood, and bone marrow.

10. A method for quantification of precursor T cells or precursor B cells, comprising: culturing cells in the detection method according to any one of Claims 1 to 9 by a limiting dilution method; analyzing the cells proliferated during the culture; and determining a ratio of cells where appearance of precursor T cells or precursor B cells is not detected.

11. A method for evaluation of property of a hematopoietic precursor cell in a transplantation source, comprising quantifying the precursor T cells or precursor B cells detected in the detection method according to any one of Claims 1 to 9.

12. A method for evaluation of property of a hematopoietic precursor cell in a transplantation source according to Claim 11, which is used for determining frequency of precursor T cells or precursor B cells per monocyte.

13. A method for evaluation of property of a hematopoietic precursor cell in a transplantation source according to Claim 11 or 12, further comprising quantifying erythrocyte and granulocyte lineage precursor cells by in vitro colony formation.

14. A transplantation source classified depending on a disease to be treated after evaluating property of a hematopoietic precursor cell by the method according to any one of Claims 11 to 13.

15. A method for selection of transplantation source classified depending on a disease to be treated after evaluating the property of a hematopoietic precursor cell by a method according to any one of Claims 11 to 13.

16. A kit for evaluating a transplantation source using the detection method according to any one of Claims 1 to 9.

17. A kit for evaluating a transplantation source according to Claim 16, comprising a stromal cell line.

18. A kit for evaluating a transplantation source according to Claim 17, wherein the stromal cell line is a stroma cell line TSt-4 where DLL1 or Dll1 is forcibly expressed.

19. A stroma cell line TSt-4 where DLL1 is forcibly expressed.

20. A stroma cell line TSt-4 where DLL1 is forcibly expressed by an introduction of a gene.

21. A stromal cell line TSt-4 where DLL1 is forcibly expressed according to Claim 20, wherein the gene introduced is a DLL1 gene.

22. A stromal cell line TSt-4 where DLL1 is forcibly expressed according to Claim 21, wherein the accession number is FERM BP-10375.


SEQUENCE LISTING

<110> Nihon University and RIKEN

<120> Detection method of human progenitors generating T
      cells and B cells.

<130> P/60652.ep01

<140> EP 06810651.7
<141> 2006-09-27

<150> JP 20050286289
<151> 2005-09-30

<160> 4

<170> PatentIn Ver. 2.1

<210> 1
<211> 2169
<212> DNA
<213> mouse

<220>
<223> Inventor: Katsura, Yoshimoto; Mugishima, Hideo; Kato, Maiko; Kawamoto
Hiroshi.


<400> 1
   atgggccgtc ggagcgcgct agcccttgcc gtggtctctg ccctgctgtg ccaggtctgg 60
   agctccggcg tatttgagct gaagctgcag gagttcgtca acaagaaggg gctgctgggg 120
   aaccgcaact gctgccgcgg gggctctggc ccgccttgcg cctgcaggac cttctttcgc 180
   gtatgcctca agcactacca ggccagcgtg tcaccggagc caccctgcac ctacggcagt 240
   gctgtcacgc cagtgctggg tgtcgactcc ttcagcctgc ctgatggcgc aggcatcgac 300
   cccgccttca gcaaccccat ccgattcccc ttcggcttca cctggccagg taccttctct 360
   ctgatcattg aagccctcca tacagactct cccgatgacc tcgcaacaga aaacccagaa 420
   agactcatca gccgcctgac cacacagagg cacctcactg tgggagaaga atggtctcag 480
   gaccttcaca gtagcggccg cacagacctc cggtactctt accggtttgt gtgtgacgag 540
   cactactacg gagaaggttg ctctgtgttc tgccgacctc gggatgacgc ctttggccac 600
   ttcacctgcg gggacagagg ggagaagatg tgcgaccctg gctggaaagg ccagtactgc 660
   actgacccaa tctgtctgcc agggtgtgat gaccaacatg gatactgtga caaaccaggg 720
   gagtgcaagt gcagagttgg ctggcagggc cgctactgcg atgagtgcat ccgataccca 780
   ggttgtctcc atggcacctg ccagcaaccc tggcagtgta actgccagga aggctggggg 840
   ggccttttct gcaaccaaga cctgaactac tgtactcacc ataagccgtg caggaatgga 900
   gccacctgca ccaacacggg ccaggggagc tacacatgtt cctgccgacc tgggtataca 960
   ggtgccaact gtgagctgga agtagatgag tgtgctccta gcccctgcaa gaacggagcg 1020
   agctgcacgg accttgagga cagcttctct tgcacctgcc ctcccggctt ctatggcaag 1080
   gtctgtgagc tgagcgccat gacctgtgca gatggccctt gcttcaatgg aggacgatgt 1140
   tcagataacc ctgacggagg ctacacctgc cattgcccct tgggcttctc tggcttcaac 1200
   tgtgagaaga agatggatct ctgcggctct tccccttgtt ctaacggtgc caagtgtgtg 1260
   gacctcggca actcttacct gtgccggtgc caggctggct tctccgggag gtactgcgag 1320
   gacaatgtgg atgactgtgc ctcctccccg tgtgcaaatg ggggcacctg ccgggacagt 1380
   gtgaacgact tctcctgtac ctgcccacct ggctacacgg gcaagaactg cagcgcccct 1440
   gtcagcaggt gtgagcatgc accctgccat aatggggcca cctgccacca gaggggccag 1500
   cgctacatgt gtgagtgcgc ccagggctat ggcggcccca actgccagtt tctgctccct 1560
   gagccaccac cagggcccat ggtggtggac ctcagtgaga ggcatatgga gagccagggc 1620
   gggcccttcc cctgggtggc cgtgtgtgcc ggggtggtgc ttgtcctcct gctgctgctg 1680
   ggctgtgctg ctgtggtggt ctgcgtccgg ctgaagctac agaaacacca gcctccacct 1740
   gaaccctgtg ggggagagac agaaaccatg aacaacctag ccaattgcca gcgcgagaag 1800
   gacgtttctg ttagcatcat tggggctacc cagatcaaga acaccaacaa gaaggcggac 1860
   tttcacgggg accatggagc cgagaagagc agctttaagg tccgataccc cactgtggac 1920
   tataacctcg ttcgagacct caagggagat gaagccacgg tcagggatac acacagcaaa 1980
   cgtgacacca agtgccagtc acagagctct gcaggagaag agaagatcgc cccaacactt 2040
   aggggtgggg agattcctga cagaaaaagg ccagagtctg tctactctac ttcaaaggac 2100
   accaagtacc agtcggtgta tgttctgtct gcagaaaagg atgagtgtgt tatagcgact 2160
   gaggtgtaa                                                         2169

<210> 2
<211> 2172
<212> DNA
<213> human

<220>
<223> Inventor: Katsura, Yoshimoto; Mugishima, Hideo; Kato, Maiko; Kawamoto,
Hiroshi.

<400> 2
   atgggcagtc ggtgcgcgct ggccctggcg gtgctctcgg ccttgctgtg tcaggtctgg 60
   agctctgggg tgttcgaact gaagctgcag gagttcgtca acaagaaggg gctgctgggg 120
   aaccgcaact gctgccgcgg gggcgcgggg ccaccgccgt gcgcctgccg gaccttcttc 180
   cgcgtgtgcc tcaagcacta ccaggccagc gtgtcccccg agccgccctg cacctacggc 240
   agcgccgtca cccccgtgct gggcgtcgac tccttcagtc tgcccgacgg cgggggcgcc 300
   gactccgcgt tcagcaaccc catccgcttc cccttcggct tcacctggcc gggcaccttc 360
   tctctgatta ttgaagctct ccacacagat tctcctgatg acctcgcaac agaaaaccca 420
   gaaagactca tcagccgcct ggccacccag aggcacctga cggtgggcga ggagtggtcc 480
   caggacctgc acagcagcgg ccgcacggac ctcaagtact cctaccgctt cgtgtgtgac 540
   gaacactact acggagaggg ctgctccgtt ttctgccgtc cccgggacga tgccttcggc 600
   cacttcacct gtggggagcg tggggagaaa gtgtgcaacc ctggctggaa agggccctac 660
   tgcacagagc cgatctgcct gcctggatgt gatgagcagc atggattttg tgacaaacca 720
   ggggaatgca agtgcagagt gggctggcag ggccggtact gtgacgagtg tatccgctat 780
   ccaggctgtc tccatggcac ctgccagcag ccctggcagt gcaactgcca ggaaggctgg 840
   gggggccttt tctgcaacca ggacctgaac tactgcacac accataagcc ctgcaagaat 900
   ggagccacct gcaccaacac gggccagggg agctacactt gctcttgccg gcctgggtac 960
   acaggtgcca cctgcgagct ggggattgac gagtgtgacc ccagcccttg taagaacgga 1020
   gggagctgca cggatctcga gaacagctac tcctgtacct gcccacccgg cttctacggc 1080
   aaaatctgtg aattgagtgc catgacctgt gcggacggcc cttgctttaa cgggggtcgg 1140
   tgctcagaca gccccgatgg agggtacagc tgccgctgcc ccgtgggcta ctccggcttc 1200
   aactgtgaga agaaaattga ctactgcagc tcttcaccct gttctaatgg tgccaagtgt 1260
   gtggacctcg gtgatgccta cctgtgccgc tgccaggccg gcttctcggg gaggcactgt 1320
   gacgacaacg tggacgactg cgcctcctcc ccgtgcgcca acgggggcac ctgccgggat 1380
   ggcgtgaacg acttctcctg cacctgcccg cctggctaca cgggcaggaa ctgcagtgcc 1440
   cccgtcagca ggtgcgagca cgcaccctgc cacaatgggg ccacctgcca ccagaggggc 1500
   cacggctatg tgtgcgaatg tgcccgaagc tacgggggtc ccaactgcca gttcctgctc 1560
   cccgagctgc ccccgggccc agcggtggtg gacctcactg agaagctaga gggccagggc 1620
   gggccattcc cctgggtggc cgtgtgcgcc ggggtcatcc ttgtcctcat gctgctgctg 1680
   ggctgtgccg ctgtggtggt ctgcgtccgg ctgaggctgc agaagcaccg gcccccagcc 1740
   gacccctgcc ggggggagac ggagaccatg aacaacctgg ccaactgcca gcgtgagaag 1800
   gacatctcag tcagcatcat cggggccacg cagatcaaga acaccaacaa gaaggcggac 1860
   ttccacgggg accacagcgc cgacaagaat ggcttcaagg cccgctaccc agcggtggac 1920
   tataacctcg tgcaggacct caagggtgac gacaccgccg tcagggacgc gcacagcaag 1980
   cgtgacacca agtgccagcc ccagggctcc tcaggggagg agaaggggac cccgaccaca 2040
   ctcaggggtg gagaagcatc tgaaagaaaa aggccggact cgggctgttc aacttcaaaa 2100
   gacaccaagt accagtcggt gtacgtcata tccgaggaga aggatgagtg cgtcatagca 2160
   actgaggtgt aa 2172

<210> 3
<211> 21
<212> DNA
<213> Artificial Sequence

<220>
<223> Description of Artificial Sequence: Primer

<220>
<223> Inventor: Katsura, Yoshimoto; Mugishima, Hideo; Kato, Maiko; Kawamoto,
Hiroshi.

<400> 3
tggtggtctc tcccaggctc t 21

<210> 4

<211> 21
<212> DNA
<213> Artificial Sequence

<220>
<223> Description of Artificial Sequence: Primer

<220>
<223> Inventor: Katsura, Yoshimoto; Mugishima, Hideo; Kato, Maiko; Kawamoto,
Hiroshi.

<400> 4
ccagctgtcc agccttgact t                                                21